# EUROPÄISCHE PATENTSCHRIFT

(11) **EP 0 885 645 B1**
(45) Veröffentlichungstag und Bekanntmachung des Hinweises auf die Patenterteilung: **05.01.2005**
(21) Anmeldenummer: 98106126.0
(22) Anmeldetag: 03.04.1998
(51) Int. Cl.: B01D 53/04, A61M 16/00, A61M 16/10

(54) **Sauerstoffkonzentrator**
Oxygen concentrator
Concentrateur d'oxygène

(30) Priorität: 17.06.1997 DE 19725632
(43) Veröffentlichungstag der Anmeldung: 23.12.1998
(73) Patentinhaber: GOTTLIEB WEINMANN GERÄTE FÜR MEDIZIN UND ARBEITSSCHUTZ GMBH & CO., 22525 Hamburg (DE)
(72) Erfinder: Ebers, Manfred, 22457 Hamburg (DE); Willimczik, Bernd, 22880 Wedel (DE); Delin, Heinz, 25451 Quickborn (DE)
(74) Vertreter: Hansmann, Dierk, Dipl.-Ing.

(56) Entgegenhaltungen:
- US-A- 4 342 573
- US-A- 4 378 982
- US-A- 4 511 377
- US-A- 4 826 510
- US-A- 5 112 367
- DATABASE WPI Section Ch, Week 9639 Derwent Publications Ltd., London, GB; Class E36, AN 96-388444 XP002091225 & JP 08 188403 A (OKUHASHI T) , 23. Juli 1996
- DATABASE WPI Section Ch, Week 9551 Derwent Publications Ltd., London, GB; Class E36, AN 95-399629 XP002091226 & JP 07 275632 A (TEIJIN LTD) , 24. Oktober 1995

## Beschreibung

Die Erfindung betrifft einen Sauerstoffkonzentrator zur Verwendung im Bereich der medizinischen Beatmungstherapie, der als Bauelemente mindestens einen von einem Gehäuse umgebenen Kompressor sowie mindestens ein vom Kompressor gespeistes und innerhalb des Gehäuses angeordnetes Filterelement aufweist, das Stickstoff zurückhält und für Sauerstoff durchlässig ist, und bei dem innerhalb des Gehäuses ein separater Kompressorraum für den Kompressor angeordnet ist und bei dem der Kompressor lose auf Lagerblöcken innerhalb des Kompressorraumes aufgestellt ist.

Derartige Sauerstoffkonzentratoren werden im Bereich der Medizintechnik verwendet, um Patienten mit Atemluft zu versorgen, die einen gegenüber der Umgebungsluft erhöhten Sauerstoffanteil aufweist. Im Hinblick auf zunehmend steigende Anforderungen an die Vereinfachung von Montage- und Demontagevorgängen sowie bezüglich von Verbesserungen der Schall- und Vibrationsdämpfung können die bislang bekannten Geräte nicht mehr alle an sie gestellten Anforderungen erfüllen.

Ähnliche Anforderungen im Bereich der Herstellung von Geräten der Datenverarbeitung konnten gemäß der EP-B-0 546 211 dadurch erfüllt werden, daß ein Computerchassis aus einem Schaumstoff hergestellt wurde, in dem die verwendeten elektronischen Komponenten durch Formschluß gehalten werden. Eine unmittelbare Anwendung dieses Lösungsansatzes im Bereich der Medizintechnik erweist sich jedoch nicht als ausreichend.

Aus der US-A-46 67 669 ist bereits ein Sauerstoffkonzentrator bekannt, der eine Mehrzahl von Bauteilen aufweist und bei dem über eine vorgesehene Steuerung Einatmungsphasen sowie Ausatmungsphasen beeinflußt werden.

In der DE-A-22 44 887 wird eine Atemmaske beschrieben, bei der ein schaumartiges Material verwendet wird, um eine Konturanpassung an das Gesicht des Patienten vorzunehmen. Durch die Konturanpassung kann eine hohe Abdichtwirkung erreicht werden.

Aus der US-A-48 25 863 ist es bekannt, ein Beatmungsgerät derart zu konstruieren, daß ein Gehäuse aus zwei konzentrischen Gehäuseteilen ausgebildet ist. Die Gehäusehälften sind von einem geschäumten Material miteinander verbunden, ohne daß dieses Material jedoch Bauteile innerhalb des Gehäuses haltert.

Die DE 100 95 40 344 A1 beschreibt ein Beatmungsgerät, das einen speziellen Überdruckverlauf bei der Patientenatmung generiert. Während der Überdruckphasen kann der Patient eine erhöhte Sauerstoffmenge aufnehmen. Der Druckverlauf wird durch ein geeignet ansteuerbares Gebläse erzeugt, das von einem Motor angetrieben ist.

Aus der US-A-4,378,982 ist es bereits bekannt, einen Sauerstoffkonzentrator mit einem Kompressor sowie einem Filterelement zu konstruieren, wobei das Filterelement Stickstoff zurückhält und für Sauerstoff durchlässig ist. Der Kompressor ist innerhalb eines separaten Kompressorraumes des Gehäuses angeordnet und innerhalb des Kompressorraumes lose auf Lagerblöcken aufgestellt. Das Gehäuse ist zur Geräuschdämmung mit Platten aus geschäumten Material versehen.

Aus der US-A-4,342,573 ist ein weiterer Sauerstoffkonzentrator bekannt, der mit einem Kompressor versehen ist. Der Kompressor ist in einem separaten Kompressorraum angeordnet. Zur Geräuschdämmung ist der Kompressorraum mit Platten aus einem geschäumten Material ausgekleidet, um eine Schalldämmung zu erreichen.

Aufgabe der vorliegenden Erfindung ist es, einen Sauerstoffkonzentrator der einleitend genannten Art derart auszubilden, daß verbesserte Eigenschaften bei der Produktion und der späteren Nutzung bereitgestellt werden.

Diese Aufgabe wird erfindungsgemäß dadurch gelöst, daß mindestens einige der innerhalb des Gehäuses angeordneten Bauelemente von einem geschäumten Träger gehaltert sind, daß zusätzlich mindestens zwei weitere geschäumte Abdeckelemente gegen den Träger zur Umschließung der Bauelemente grenzen und daß sich der Träger im wesentlichen vertikal erstreckt.

Durch die Verwendung des Trägers aus einem geschäumten Material wird es auch im medizintechnischen Bereich ermöglicht, Bauelemente lediglich durch Formschluß zu haltern. Aufgrund der speziellen medizintechnischen Anforderungen sowie der konstruktiven Besonderheiten von Sauerstoffkonzentratoren wurde jedoch festgestellt, daß zur Abdeckung und zuverlässigen Halterung der Bauelemente mindestens zwei weitere geschäumte Abdeckelemente erforderlich sind, die gegen den Träger grenzen. Der Träger kann hierdurch im Bereich von zwei aneinander abgewandter Montageflächen mit Bauelementen bestückt werden, so daß mindestens zwei voneinander trennbare Montagebereiche bereitgestellt werden.

Eine besonders gute Schall- und Vibrationsdämmung wird dadurch hervorgerufen, daß innerhalb des Gehäuses ein separater Kompressorraum für den Kompressor angeordnet ist.

Zur Gewährleistung einer hohen Nutzluftqualität wird vorgeschlagen, daß getrennte Strömungswege für Kühlluft und Nutzluft angeordnet sind.

Eine besonders gute Montage- und Servicefreundlichkeit kann dadurch hervorgerufen werden, daß der Träger mit drei separaten Abdeckelementen versehen ist.

Zur Unterstützung eines gleichmäßigen Betriebes mit zyklischen Regenerationsphasen wird vorgeschlagen, daß der Träger zwei Filterelemente haltert.

Eine langfristige Betriebsfähigkeit kann dadurch unterstützt werden, daß innerhalb der Filterelemente Molekularfilter mit granulatartigem Filtermaterial zur Stickstoffanlagerung angeordnet sind. Insbesondere sind Granulate auf Basis von Zeolithen verwendbar.

Zur Realisierung einer Ansteuerung wird vorgeschlagen, daß die Filterelemente von Ventilen angesteuert sind.

Eine Filterregeneration kann dadurch durchgeführt werden, daß zur Regeneration des Filtermaterials eine zeitweilige Rückströmung zur Stickstoffausspülung vorgesehen ist.

Zur Bereitstellung einer ausreichenden Versorgungssicherheit mit gleichbleibendem Druckniveau wird vorgeschlagen, daß die Filterelemente mit einem Sammelbehälter verbunden sind.

Eine einfache Montage und Demontage wird dadurch unterstützt, daß der Träger und die Abdeckelemente von mindestens einem Riemen miteinander verspannt sind.

Eine weitere Reduktion von mechanischen Verbindungsteilen kann dadurch erfolgen, daß das Gehäuse über einen Handgriff mit dem Träger verbunden ist.

Ein sehr effektiver Ablauf des Regenerationsvorganges im Filterbereich wird dadurch unterstützt, daß die Ventile mit einer Steuerung zur Vorgabe einer mindestens zeitweilig gleichzeitigen Öffnung zur Durchführung der Rückspülung verbunden sind.

Die Vibrationsdämpfung kann auch dadurch unterstützt werden, daß der Kompressor lose auf Lagerblöcken innerhalb des Kompressorraumes aufgestellt ist.

Eine weitere Verbesserung der Montagefreundlichkeit erfolgt dadurch, daß der Träger im wesentlichen lotrecht angeordnete Montageflächen aufweist, in die die zu halternden Bauelemente in horizontaler Richtung einsetzbar sind.

Zur weiteren Optimierung der Geräuschdämmung wird vorgeschlagen, daß Lüftungskanäle zur Kühlluftableitung im Bereich eines Rückenteiles nach außen geführt sind.

Eine zweckmäßige Strömungsführung besteht darin, daß mindestens einer der Luftkanäle unterhalb des Kompressors in einer Grundplatte verläuft.

Ebenfalls kann die Geräuschdämmung dadurch unterstützt werden, daß die Luftkanäle im Ausströmungsbereich in ein Ausgangslabyrinth zur Geräuschdämmung münden.

Eine zweckmäßige Materialkombination zur Bereitstellung einer ausreichenden mechanischen Festigkeit sowie einer gleichzeitigen guten Geräusch- und Vibrationsdämpfung besteht darin, daß der Träger und die Abdeckelemente aus einem Schaumstoff mit einer Materialdichte im Bereich von 40 g/l bis 60 g/l bestehen.

In der Zeichnung ist ein Ausführungsbeispiel der Erfindung schematisch dargestellt. Es zeigen:
- Fig. 1: Eine rückwärtige Ansicht des Sauerstoffkonzentrators mit abgenommenem Gehäuse und abgenommener Rückenschale,
- Fig. 2: eine perspektivische Vorderansicht des Sauerstoffkonzentrators gemäß Fig. 1 mit abgenommenem Gehäuse sowie abgenommener Vorderschale,
- Fig. 3: einen stark vergrößerten Querschnitt im Bereich eines Überganges des Gehäuses in ein Griffteil und
- Fig. 4: ein vereinfachtes pneumatisches Schaltbild der eingesetzten pneumatischen Komponenten.

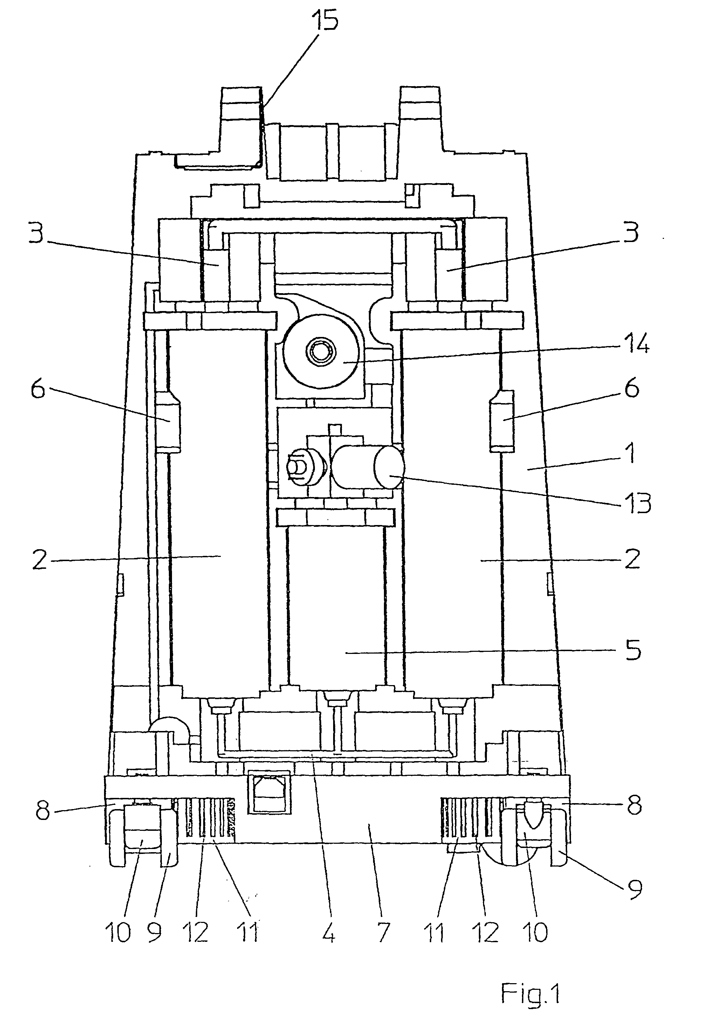

Fig. 1 zeigt in einer rückwärtigen Ansicht die Einbausituation im Bereich eines Trägers (1). Der Träger (1) haltert bezüglich ihrer Längsachsen im wesentlichen vertikal angeordnete Filterelemente (2), die bei einer Gasdurchströmung Stickstoff zurückhalten. Beispielsweise ist es möglich, die Filterelemente (2) mit einer im wesentlichen zylindrischen Gehäuseform zu versehen und die zu trennende Luft über Ventile (3) in einem Kopfbereich der Filterelemente (2) zuzuführen. Abströmende und mit Sauerstoff angereicherte Luft wird über Verbindungsleitungen (4) in den Bereich eines Sammelbehälters (5) geleitet, der ebenfalls zylindrisch konstruiert sein kann.

Die Filterelemente (2) können derart ausgebildet sein, daß in ihrem Inneren ein granulatartiges Material angeordnet wird, an das sich Stickstoff bei einer Durchströmung anlagert und das Sauerstoff passieren läßt. Geeignet sind vor allem Materialien auf Basis von Zeolithen.

Der Träger (1) weist zur Halterung der Filterelemente (2) und des Sammelbehälters (5) muldenförmige Vertiefungen auf. Ein Festklemmen der Filterelemente (2) in den muldenförmigen Vertiefungen kann über Klemmzungen (6) erfolgen. Der Träger (1) erstreckt sich mit einer Grundfläche im wesentlichen vertikal, so daß die gehalterten Bauelemente in horizontaler Richtung eingesetzt und entnommen werden können.

In einem Bodenbereich mündet der Träger (1) in eine Grundplatte (7) ein, die vorzugsweise einteilig mit dem Träger (1) ausgebildet ist. Die Grundplatte (7) weist in Vertiefungen (8) angeordnete Räder (9) auf, die von schwenkbaren Radgestellen (10) gehaltert sind.

Durch die Grundplatte (7) hindurch erstrecken sich Lüftungskanäle (11) für Kühlluft, die im Bereich ihrer Überleitung in eine Umgebung mit einer labyrinthartigen Ausströmungsstruktur zur Geräuschminderung versehen sind.

Oberhalb des Sammelbehälters (5) ist ein Druckminderer (13) angeordnet, desweiteren ist vom Träger (1) ein Ventilator (14) zur Erzeugung einer erforderlichen Kühlluftströmung durch die Lüftungskanäle (11) hindurch gehaltert.

Die Ventile (3), die vorzugsweise als Drei-Zwei-Wegeventile ausgebildet sind, sind über Verrohrungen eingangsseitig an einen Feinfilter sowie einen vorgeschalteten Grobfilter zur Zurückhaltung von Luftverunreinigungen angeschlossen. Als Ventile (3) können beispielsweise ebenfalls 5-3-Wegeventile bzw. Ventile mit Mittelstellung verwendet werden. Ausgangsseitig weisen die Ventile (3) eine Verbindung zu Schalldämpfern auf.

Über eine geeignete Ansteuerung der Ventile (3) ist es möglich, eine abwechselnde Aktivierung der beiden Filterelemente (2) vorzunehmen. Nach einer Deaktivierung eines der Filterelemente (2) erfolgt in diesem Filterelement (2) ein partielles Rückströmen von Luft, so daß der im Filterelement (2) zurückgehaltene Stickstoff wieder ausgetragen und an die Umgebung abgegeben wird. Durch dieses Regenerationsprinzip wird nach Durchlaufen jeden alternierenden Arbeitszyklusses wieder der Ausgangszugestand bereitgestellt, so daß Wartungsarbeiten weitgehend entbehrlich sind. Insbesondere ist es nicht erforderlich, die Filterelemente (2) aufgrund zunehmender Stickstoffansammlung auszuwechseln.

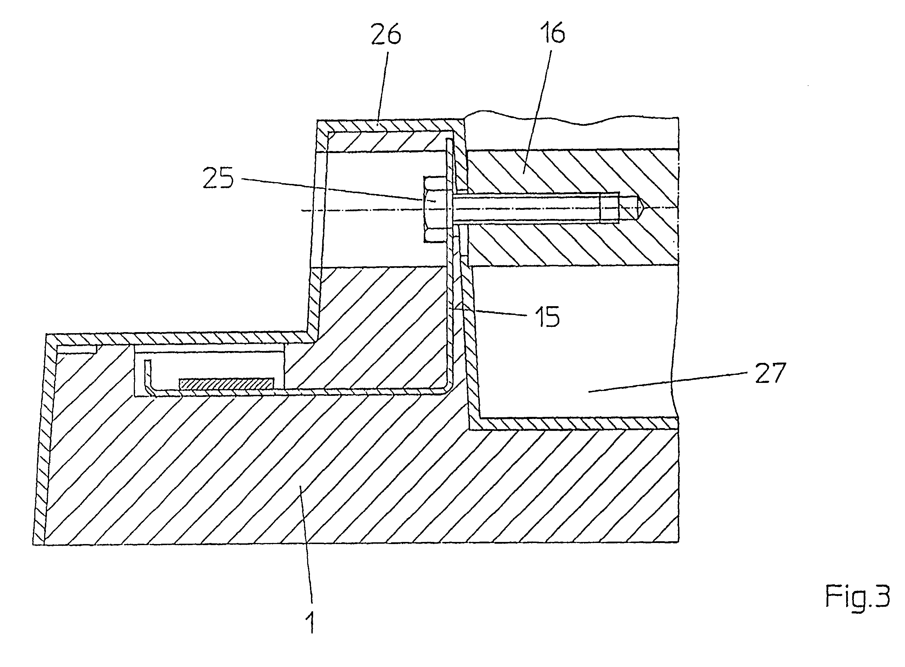

In lotrechter Richtung oben sind in den Träger (1) Haltebügel (15) eingebettet, die zur Befestigung eines in Fig. 3 dargestellten Handgriffes (16) dienen. Der Handgriff (16) wird nach einem Aufsetzen eines nicht dargestellten Gehäuses auf den Träger (1) montiert und verbindet nach einer entsprechenden Befestigung den Träger (1) mit dem äußeren Gehäuse.

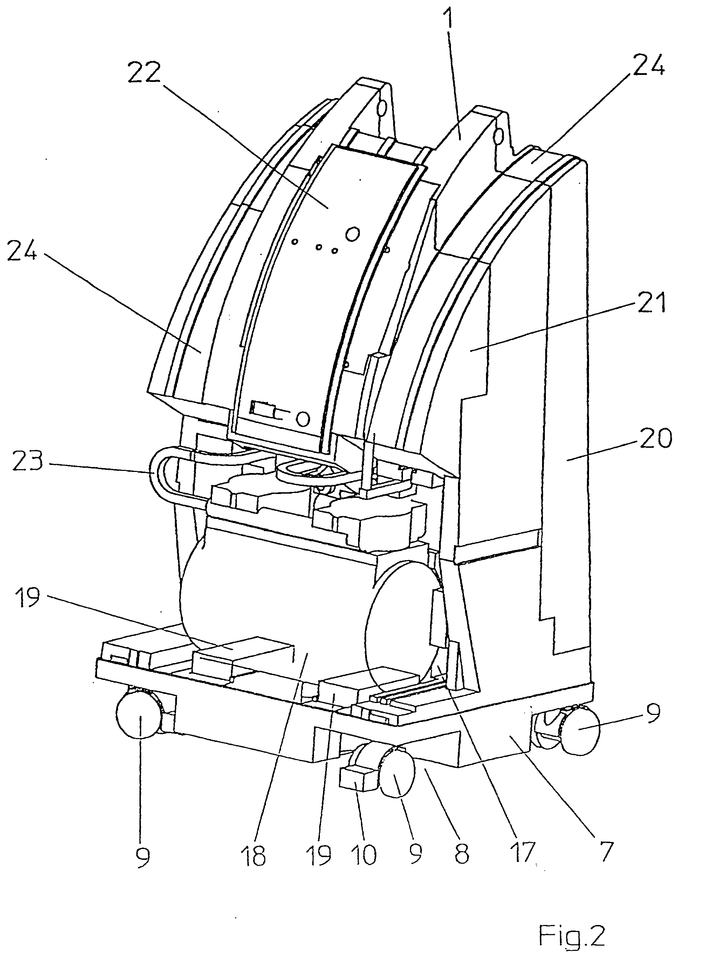

Aus der perspektivischen Darstellung in Fig. 2 ist erkennbar, daß innerhalb eines abgegrenzten Kompressorraumes (17) ein Kompressor (18) angeordnet ist. Der Kompressorraum erstreckt sich oberhalb der Grundplatte (7). Der Kompressor steht ohne separate Befestigung auf Lagerblöcken (19) und wird ebenfalls durch Formschluß fixiert.

Aus der Darstellung ist ebenfalls erkennbar, daß im rückwärtigen Teil der Träger (1) mit den gehalterten Bauelementen von einem Rückenteil (20) abgedeckt ist. Oberhalb des Kompressors (18) erstreckt sich eine vom Träger (1) abnehmbare Abdeckschale (21), die ein Bedienfeld (22) trägt.

Durch die Anordnung des Kompressors (18) in einem separaten Kompressorraum (17) und die Lagerung auf den Lagerblöcken (19) wird eine sehr gute Schall- und Vibrationsentkopplung bereitgestellt. Kühlelemente (23) werden von Kühlluft umströmt, die durch die Lüftungskanäle (11) hindurch in die Umgebung entweichen kann. Die von den Filterelementen (2) bereitgestellte und mit Sauerstoff angereicherte Luft wird in separaten Luftleitungen getrennt von der Kühlluft geführt.

Der Träger (1), die Abdeckschale (21), das Rückenteil (20) sowie ein nicht dargestelltes Abdeckelement für den Kompressorraum (17) werden von Gurten zusammengehalten, die mit Schnellverschlüssen versehen und in Vertiefungen (24) geführt sind. Es kann hierdurch eine sehr schnelle Montage und Demontage unterstützt werden.

Aus der Darstellung in Fig. 2 ist ebenfalls erkennbar, daß der Träger (1), das Rückenteil (20) die Abdeckschale (21) sowie die Verschlußschale für den Kompressorraum (17) eine stufenförmige Kontur aufweisen, um nach einem Zusammenfügen eine Selbstausrichtung durchzuführen. Es wird hierdurch eine exakt definierte und reproduzierbare Einbausituation bereitgestellt.

Aus der vergrößerten Querschnittdarstellung in Fig. 3 ist erkennbar, daß der Handgriff (16) über eine Verschraubung (25) mit dem Haltebügel (15) verbunden ist. Die Verbindung erfolgt nach einem Aufsetzen des Gehäuses (26) auf den Träger (1) und die Abdeckelemente. Durch diese Verbindung wird somit eine Fixierung des Gehäuses (26) relativ zum Träger (1) hervorgerufen. Unterhalb des Handgriffes (16) ist eine Griffmulde (27) angeordnet, um ein Tragen und Handhaben des Gerätes zu unterstützen.

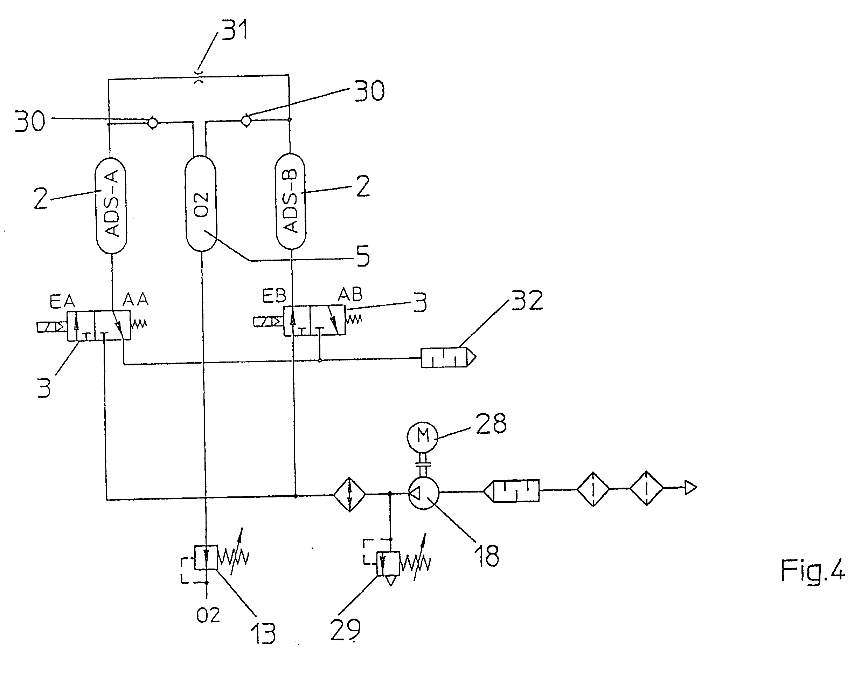

Fig. 4 zeigt den wesentlichen pneumatischen Aufbau. Der Kompressor (18) wird von einem Motor (28) angetrieben und verdichtet die zuvor einem Grobfilter und einem Feinfilter zugeleitete Umgebungsluft. Der Ausgang des Kompressors (18) ist mit einem Überdruckventil (29) verbunden und versorgt über die Ventile (3) die Filterelemente (2). Die Filterelemente (2) sind über Rückschlagventile (30) an den Sammelbehälter (5) angeschlossen. Zusätzlich ist eine Überbrückung mit einer Spüldüse (31) vorgesehen. Ein Ausgang des Sammelbehälters (5) ist an den Druckminderer (13) angeschlossen. Bei einer Rückspülung der Filterelemente (2) zum Entfernen des angelagerten Stickstoffes nach entsprechender Freischaltung der Ventile (3) erfolgt eine Schalldämpfung der abströmenden Luft über einen Abgasschalldämpfer (32).

Bei einer Durchführung der Rückspülung für die Stickstoffentfernung erfolgt eine gleichzeitige Öffnung der Ventile (3) zur Kopplung über die Spüldüse (31) für etwa eine Sekunde. Dieser Zeitraum ist in der Regel für eine Regeneration des Filtermaterials ausreichend.

Eines der Ventile (3) gibt hierbei eine Verbindung mit dem Kompressor (18) frei und das dem spülenden Filterelement (2) zugeordnete Ventil (3) führt eine Verbindung mit dem Abgasschalldämpfer (32) durch.

Eine weitere Schalldämmung kann dadurch erfolgen, daß die dem Kompressor (18) zugeführte Umgebungsluft durch zwei versetzt in den Ansaugkanal eingebrachte Schläuche hindurchströmt. Eine Schalldämpfung im Bereich der Abblasluft kann beispielsweise mit offenzelligem Schaumstoff oder Filz unterstützt werden. Die jeweilige Materialauswahl kann in Anpassung an das vorliegende Geräuschspektrum erfolgen.

Als Material für die Lagerblöcke (19) des Kompressors (18) kommt beispielsweise ein geschlossenzelliges Polyurethan in Frage. Die Lagerblöcke (19) verhindern eine direkte Übertragung der Vibrationen des Kompressors (18) auf das Schaummaterial des Trägers (1) und der verwendeten Abdeckelemente. Die Innenkontur des Kompressorraumes (17) ist durch die Dimensionierung der eingesetzten geschäumten Elemente so gewählt, daß relativ zum Kompressor (18) ein maximales Spiel von 5mm vorliegt. Hierdurch werden Relativbewegungen zwischen den Bauteilen kontrolliert und Beschädigungen bei einem Einwirken von Beschleunigungen vermieden.

Eine typische Dichte für das geschäumte Material des Trägers (1) und der Abdeckelemente beträgt 40 g/l bis 60 g/l. Als Material für diese Bauteile kann beispielsweise expandiertes Polypropylen, expandiertes Polyethylen oder Polystyrol verwendet werden.

## Patentansprüche

1. Sauerstoffkonzentrator zur Verwendung im Bereich der medizinischen Beatmungstherapie, der mindestens einen von einem Gehäuse (26) umgebenen Kompressor (18) sowie mindestens ein vom Kompressor (18) gespeistes und innerhalb des Gehäuses (26) angeordnetes Filterelement (2) aufweist, das Stickstoff zurückhält und für Sauerstoff durchlässig ist, und bei dem innerhalb des Gehäuses (26) ein separater Kompressorraum (17) für den Kompressor (18) angeordnet ist und daß der Kompressor (18) lose auf Lagerblöcken (19) innerhalb des Kompressorraumes (17) aufgestellt ist, **dadurch gekennzeichnet, daß** mindestens einige der innerhalb des Gehäuses (26) angeordneten Bauelemente von einem geschäumten Träger (1) gehaltert sind, daß zusätzlich mindestens zwei weitere geschäumte Abdeckelemente gegen den Träger (1) zur Umschließung der Bauelemente grenzen und daß sich der Träger (1) im wesentlichen vertikal erstreckt.

2. Sauerstoffkonzentrator nach Anspruch 1, **dadurch gekennzeichnet, daß** getrennte Strömungswege für Kühlluft und Nutzluft angeordnet sind.

3. Sauerstoffkonzentrator nach einem der Ansprüche 1 oder 2, **dadurch gekennzeichnet, daß** der Träger (1) mit drei separaten Abdeckelementen versehen ist.

4. Sauerstoffkonzentrator nach einem der Ansprüche 1 bis 3, **dadurch gekennzeichnet, daß** der Träger (1) zwei Filterelemente (2) haltert.

5. Sauerstoffkonzentrator nach einem der Ansprüche 1 bis 4, **dadurch gekennzeichnet, daß** innerhalb der Filterelemente (2) Molekularfilter mit granulatartigem Filtermaterial zur Stickstoffanlagerung angeordnet sind.

6. Sauerstoffkonzentrator nach einem der Ansprüche 1 bis 5, **dadurch gekennzeichnet, daß** die Filterelemente (2) von Ventilen (3) angesteuert sind.

7. Sauerstoffkonzentrator nach einem der Ansprüche 1 bis 6, **dadurch gekennzeichnet, daß** zur Regeneration des Filtermaterials eine zeitweilige Rückströmung zur Stickstoffausspülung vorgesehen ist.

8. Sauerstoffkonzentrator nach einem der Ansprüche 1 bis 7, **dadurch gekennzeichnet, daß** die Filterelemente (2) mit einem Sammelbehälter (5) verbunden sind.

9. Sauerstoffkonzentrator nach einem der Ansprüche 1 bis 8, **dadurch gekennzeichnet, daß** der Träger (1) und die Abdeckelemente von mindestens einem Riemen miteinander verspannt sind.

10. Sauerstoffkonzentrator nach einem der Ansprüche 1 bis 9, **dadurch gekennzeichnet, daß** das Gehäuse (26) über einen Handgriff (16) mit dem Träger (1) verbunden ist.

11. Sauerstoffkonzentrator nach einem der Ansprüche 1 bis 10, **dadurch gekennzeichnet, daß** die Ventile (3) mit einer Steuerung zur Vorgabe einer mindestens zeitweilig gleichzeitigen Öffnung zur Durchführung der Rückspülung verbunden sind.

12. Sauerstoffkonzentrator nach einem der Ansprüche 1 bis 11, **dadurch gekennzeichnet, daß** der Träger (1) im wesentlichen lotrecht angeordnete Montageflächen aufweist, in die die zu halternden Bauelemente in horizontaler Richtung einsetzbar sind.

13. Sauerstoffkonzentrator nach einem der Ansprüche 1 bis 12, **dadurch gekennzeichnet, daß** Lüftungskanäle (11) zur Kühlluftableitung im Bereich eines Rückenteiles (20) nach außen geführt sind.

14. Sauerstoffkonzentrator nach einem der Ansprüche 1 bis 13, **dadurch gekennzeichnet, daß** mindestens einer der Luftkanäle (11) unterhalb des Kompressors (18) in einer Grundplatte (7) verläuft.

15. Sauerstoffkonzentrator nach einem der Ansprüche 1 bis 14, **dadurch gekennzeichnet, daß** die Luftkanäle (11) im Ausströmungsbereich in ein Ausgangslabyrinth zur Geräuschdämmung münden.

16. Sauerstoffkonzentrator nach einem der Ansprüche 1 bis 15, **dadurch gekennzeichnet, daß** der Träger (1) und die Abdeckelemente aus einem Schaumstoff mit einer Materialdichte im Bereich von 40 g/l bis 60 g/l bestehen.

## Claims

1. Oxygen concentrator for use in the field of medical respiration therapy which has at least one compressor (18) surrounded by a housing (26) and at least one filter element (2) fed by the compressor (18) and arranged inside the housing (26), which traps nitrogen and is permeable to oxygen, and in which a separate compressor chamber (17) for the compressor (18) is arranged inside the housing (26) and that the compressor (18) is mounted loosely on bearing blocks (19) inside the compressor chamber (17), **characterised in that** at least some of the structural elements arranged inside the housing (26) are supported by a foamed support (1), that additionally at least two other foamed cover elements abut the support (1) to enclose the structural elements and that the support (1) extends essentially vertically.

2. Oxygen concentrator according to Claim 1, **characterised in that** separate flow routes are arranged for cooling air and effective air.

3. Oxygen concentrator according to one of Claims 1 or 2, **characterised in that** the support (1) is provided with three separate cover elements.

4. Oxygen concentrator according to one of Claims 1 to 3, **characterised in that** the support (1) supports two filter elements (2).

5. Oxygen concentrator according to one of Claims 1 to 4, **characterised in that** molecular filters with granular filter material to take up the nitrogen are arranged inside the filter elements (2).

6. Oxygen concentrator according to one of Claims 1 to 5, **characterised in that** the filter elements (2) are controlled by valves (3).

7. Oxygen concentrator according to one of Claims 1 to 6, **characterised in that** a temporary back-flow to the nitrogen flush is provided to regenerate the filter material.

8. Oxygen concentrator according to one of Claims 1 to 7, **characterised in that** the filter elements (2) are connected to a collecting tank (5).

9. Oxygen concentrator according to one of Claims 1 to 8, **characterised in that** the support (1) and the cover elements are braced together by at least one belt.

10. Oxygen concentrator according to one of Claims 1 to 9, **characterised in that** the housing (26) is connected to the support (1) by means of a handle (16).

11. Oxygen concentrator according to one of Claims 1 to 10, **characterised in that** the valves (3) are connected to a control to provide an at least temporary simultaneous opening to carry out backflushing.

12. Oxygen concentrator according to one of Claims 1 to 11, **characterised in that** the support (1) has assembly areas arranged essentially perpendicular into which the structural elements to be supported can be placed in a horizontal direction.

13. Oxygen concentrator according to one of Claims 1 to 12, **characterised in that** the ventilation ducts (11) are taken outside to draw off cooling air in the area of a back part (20).

14. Oxygen concentrator according to one of Claims 1 to 13, **characterised in that** at least one of the air ducts (11) passes below the compressor (18) in a base plate (7).

15. Oxygen concentrator according to one of Claims 1 to 14, **characterised in that** the air ducts (11) lead in the discharge area into an outlet labyrinth to dampen noise.

16. Oxygen concentrator according to one of Claims 1 to 15, **characterised in that** the support (1) and the cover elements consist of a foam with a material density in the range of 40 g/l to 60 g/l.

## Revendications

1. Concentrateur d'oxygène à utiliser dans le domaine de la thérapie respiratoire médicale, qui présente au moins un compresseur (18) entouré d'un boîtier (26), ainsi qu'au moins un élément de filtration (2) alimenté par le compresseur et agencé à l'intérieur du boîtier (26), qui retient l'azote et est perméable pour l'oxygène, et dans lequel une chambre de compresseur séparée (17) pour le compresseur (18) est agencée à l'intérieur du boîtier (26), et en ce que le compresseur (18) est monté de manière lâche sur des blocs de support (19) à l'intérieur de la chambre de compresseur (17), **caractérisé en ce qu'**au moins certains des composants agencés à l'intérieur du boîtier (26) sont maintenus par un support (1) en matériau expansé, **en ce qu'**au moins deux éléments de recouvrement supplémentaires en matériau expansé s'étendent contre le support (1) pour entourer les composants, et **en ce que** le support (1) s'étend sensiblement verticalement.

2. Concentrateur d'oxygène selon la revendication 1, **caractérisé en ce que** des chemins d'écoulement séparés sont agencés pour l'air de refroidissement et pour l'air utile.

3. Concentrateur d'oxygène selon l'une des revendications 1 et 2, **caractérisé en ce que** le support (1) est pourvu de trois éléments de recouvrement séparés.

4. Concentrateur d'oxygène selon l'une des revendications 1 à 3, **caractérisé en ce que** le support (1) contient deux éléments de filtration (2).

5. Concentrateur d'oxygène selon l'une des revendications 1 à 4, **caractérisé en ce que** des filtres moléculaires avec un matériau de filtration granulaire pour l'absorption de l'azote, sont agencés à l'intérieur des éléments de filtration (2).

6. Concentrateur d'oxygène selon l'une des revendications 1 à 5, **caractérisé en ce que** les éléments de filtration (2) sont commandés par des soupapes (3).

7. Concentrateur d'oxygène selon l'une des revendications 1 à 6, **caractérisé en ce qu'**un écoulement en sens inverse temporaire pour l'élimination de l'azote est prévu pour la régénération du matériau de filtration.

8. Concentrateur d'oxygène selon l'une des revendications 1 à 7, **caractérisé en ce que** les éléments de filtration (2) sont reliés à un réservoir commun (5).

9. Concentrateur d'oxygène selon l'une des revendications 1 à 8, **caractérisé en ce que** le support (1) et les éléments de recouvrement sont calés ensemble par au moins une lanière.

10. Concentrateur d'oxygène selon l'une des revendications 1 à 9, **caractérisé en ce que** le boîtier (26) est relié au support (1) par l'intermédiaire d'une poignée (16).

11. Concentrateur d'oxygène selon l'une des revendications 1 à 10, **caractérisé en ce que** les soupapes (3) sont reliées à une commande pour assurer une ouverture simultanée au moins temporaire pour effectuer le rinçage.

12. Concentrateur d'oxygène selon l'une des revendications 1 à 11, **caractérisé en ce que** le support (1) présente des surfaces de montage agencées sensiblement verticalement, dans lesquelles les composants à maintenir peuvent être placés dans la direction horizontale.

13. Concentrateur d'oxygène selon l'une des revendications 1 à 12, **caractérisé en ce que** des canaux de ventilation (11) pour l'amenée d'air de refroidissement sont conduits vers l'extérieur dans la région d'une pièce de dos (20).

14. Concentrateur d'oxygène selon l'une des revendications 1 à 13, **caractérisé en ce qu'**au moins l'un des canaux de ventilation (11) s'étend en dessous du compresseur (18), dans une plaque de base (7).

15. Concentrateur d'oxygène selon l'une des revendications 1 à 14, **caractérisé en ce que** les canaux de ventilation (11) débouchent, dans la région de décharge du flux, dans un labyrinthe de sortie pour l'amortissement du bruit.

16. Concentrateur d'oxygène selon l'une des revendications 1 à 15, **caractérisé en ce que** le support (1) et les éléments de recouvrement sont fait dans un matériau expansé avec une densité dans l'intervalle de 40 g/l à 60 g/l.
